# EUROPEAN PATENT APPLICATION

(11) **EP 4 112 121 A1**
(43) Date of publication of application: **04.01.2023**
(21) Application number: 21784764.9
(22) Date of filing: 08.04.2021
(51) Int. Cl.: A61N 5/06, A61F 9/04

(54) **LED ILLUMINATOR AND COMPLEXION RECOVERY METHOD USING SAME**

(30) Priority: 08.04.2020 KR 20200043021
(71) Applicant: Koreatechnics Co., Ltd., Seoul 06103 (KR)
(72) Inventor: LEE, Dong Yol, Seoul 06793 (KR)
(74) Representative: Plasseraud IP
(86) International application number: PCT/KR2021/004454
(87) International publication number: WO 2021/206490

(57) **Abstract**

The present invention relates to an LED illuminator including a face lens, and a complexion recovery method using same. Specifically, the present invention relates to: an LED illuminator including a face lens for controlling the direction and intensity of light radiated from an LED light source; and a method for alleviating skin aging and wrinkles by using same.

## Description

### [Technical Field]

This application claims the benefit of priority to Korean Patent Application No. 2020-0043021 filed on April 8, 2020 and Korean Patent Application No. 2021-0046164 filed on April 8, 2021, the disclosures of which are incorporated herein by reference in their entireties.

The present invention relates to an LED irradiator and a facial recovery method using the same. More particularly, the present invention relates to an LED irradiator capable of adjusting the direction and intensity of light radiated from an LED light source and a skin aging and wrinkles remedy method using the same.

### [Background Art]

Light irradiation therapy or light therapy is a technique that has been used for a long time in order to treat various diseases. Since thousands of years ago, skin disease has been treated using sunlight. In recent years, light irradiation therapy has attracted great attention in the field of facial recovery or regeneration.

Light irradiation therapy, which uses various light sources, such as sunlight, laser, fluorescent lamps, UV, and LEDs, is performed based on the principle by which light accelerates biochemical reaction in the skin. Light irradiation therapy is a kind of medical technique that treats or restores injured skin through selective regeneration or destruction of skin tissue.

Light irradiation therapy started to be systematically studied by Niels Ryberg Finsen, a Nobel prize laureate in medicine, in 1903, and has been rapidly developed since the 1970s. After research presentation that low level laser therapy (LLLT) remedies skin wrinkles in 2005, light irradiation skin care/medical equipment for home use appeared. A recent product that has led to the popularization of LLLT on a wide scale is an LED mask product that directly irradiates light to facial skin using LEDs.

In Korea, many LED mask products have been released. However, LED masks still need to be verified in terms of usability and effectiveness and have many technical problems to be solved.

First, it is not possible to provide a uniform amount of light to the face. In current LED masks, LEDs are positioned or arranged without consideration of the facial shapes of users, and optical design for uniform irradiation is insufficient. There occurs a case in which more light than necessary is radiated to a specific region due to overlapping of light sources or the amount of light that is radiated is insufficient due to a large distance between the light sources. In addition, there occurs a problem in which the distance between the light source and the skin is not uniform, and therefore the amount of light that reaches the skin by light diffusion is not uniform. It is necessary to radiate a uniform amount of light to the skin in consideration of the position or arrangement of the LEDs, complexity of the facial shape, diffusion of the light source, etc.

Second, it is not possible to provide customized light irradiation therapy based on position of the facial skin. Light radiated from the LEDs reaches a dermis layer via a stratum corneum layer of the skin, and accelerates biochemical reaction in the skin. In the facial skin, various types of skin, such as an eyebrow, on which sebaceous gland vigorously acts, and a part around the lips, particularly including an eye periphery having the thinnest stratum corneum layer, are present. Detailed design on the kind and wavelength of a light source and a lens based on skin science for detailed customized light irradiation considering skin conditions is very insufficient.

Third, it is possible to improve a method of preventing direct radiation of light from an LED light source to an eyeball. The typical side effect of an LED mask is "eye burns". When the amount of light is increased in order to maximize the therapy effect, strong light may be directly radiated to the eyeball, whereby eye burns may occur. When the amount of light is decreased in order to avoid this, the light irradiation therapy effect may be reduced by half. The current method of blocking light radiated to the eyeball is a method of continuously pressing the periphery of the eyeball using silicone having the shape of swimming goggles. If this method is used, however, a user may feel uncomfortable or may have a headache due to excessive pressing. The periphery of the eye, which is most easily wrinkled, is directly pressed, whereby the effect is rather contrary to skin regeneration/recovery. In addition, there is a shortcoming in that it is not possible to directly irradiate an infraorbital margin, which may most need light irradiation therapy.

Patent Document 1 relates to an LED mask device, wherein a plurality of LEDs configured to emit infrared light to a face of a user is installed in a mask having an opening hole, a protection means configured to prevent the introduction of light from the LEDs to eyes is formed at an inner surface of the mask inside the opening hole, and a head immobilizer configured to prevent the LEDs installed in the mask from directly contacting a facial skin is coupled to an upper surface of the mask; however, there are problems caused by pressing the periphery of the eye, nonuniform irradiation, and uniform amount of light, not customized amount of light.

Patent Document 2 relates to an LED mask capable of radiating LED light to a face to achieve skin care, whitening, and sterilization effects, and more particularly to an LED mask capable of three-dimensionally irradiating the face with light, irradiating the skin around the eyes with light while preventing dazzling as the result of the LED light being introduced into the eyes, and preventing foreign body sensation and skin injury as the result of the skin contacting a light blocking wall configured to prevent dazzling; however, there are problems in that the eye periphery is pressed and light is radiated without consideration of the skin around eyes, which is relatively weak facial skin.

Patent Document 3 relates to an LED mask device capable of complexly performing face and scalp care, wherein skin care suitable for the face and the scalp is performed using both of a visible LED element and a near-infrared LED element; however, there are problems in that uniform irradiation and customized amount of light are not suggested and it is not possible to prevent direct radiation of light to an eyeball.

Patent Document 4 relates to an LED mask system having an ion introduction function, the LED mask system including a mask unit configured to be worn on a face of a user, an LED unit disposed at the mask unit to radiate LED light to the face of the user, an ion introduction unit disposed at the mask unit so as to contact a facial skin of the user, a counter electrode unit electrically connected to the ion introduction unit, the counter electrode unit exhibiting positive polarity, the counter electrode unit being configured to allow negative ions to concentrate on the ion introduction unit, and a power supply unit configured to supply power to the LED unit and the ion introduction unit, wherein LED light is radiated to the skin of the user, and at the same time ingredients of a hydrogel mask attached to the face of the user are absorbed into the facial skin of the user using the ion introduction unit; however, there are problems in that uniform irradiation and customized amount of light are not suggested and an eye periphery is pressed.

Patent Document 5 relates to an LED mask device capable of radiating light having a wavelength range set for each facial region, and more particularly to an LED mask device capable of radiating light having a wavelength range corresponding to a mode set for a facial region-specific user when LED light effective for skin care and skin disease treatment is uniformly radiated to the face. The wavelength range may be adjusted for each region, and irradiation may be performed; however, the construction capable of performing uniform irradiation for each region is not provided. If light is actually radiated from the LED disposed as in Patent Document 5, light from the central part of the LED is bright, and light from the peripheral part of the LED is dark, and therefore spots may appear as the result of continuous use, whereby nonuniformity may occur.

Patent Document 6 relates to a beauty mask having an LED patch. An object of this invention is to provide skin treatment or beauty equipment using LED light in the form of a mask, wherein an element configured to radiate LED light is provided in the mask in the form of a patch, the patch includes a transparent flexible pad and an LED module coupled to each other, and the patch is detachably attached to an arbitrary point of the mask; however, there are problems in that the construction capable of performing uniform irradiation in a region in which the patch is attached is not provided and no light is radiated to an empty space between patches, whereby the nonuniform effect due to long-term exposure occurs.

An LED mask of Patent Document 7 includes a mask main body formed so as to cover a face of a wearer, a light source unit installed at the mask main body, the light source unit being configured to emit light having a predetermined wavelength range, and a fixing unit configured to fix the mask main body to the face of the wearer, wherein the mask main body is provided with a light guide plate configured to allow light emitted from the light source unit to move therethrough and to guide the light so as to be uniformly radiated toward the face of the wearer; however, if pattern protrusions are used, a shaded region that does not correspond to the entire region of the face, which is curved, occurs, and design for uniform irradiation is substantially impossible due to curvature of the face, and the light source is disposed only at a part of the mask, whereby there is a concern of low-temperature burning due to partial overheating.

Patent Document 8 relates to a beauty mask configured to allow a medicinal fluid having a predetermined efficacy to permeate a face in order to improve the skin, the beauty mask including inner and outer skins formed so as to have contours capable of being brought into tight contact with at least the face, a space configured to receive a medicinal fluid therein as the result of the contours of the inner and outer skins being integrally joined to each other, an injection pipe protruding from the outer skin such that the space is filled with the medicinal fluid, a transparent tube-shaped wearing unit having a stopper configured to be selectively detachably attached to the injection pipe in order to open and close the space, the wearing unit being capable of being contracted and relaxed, and a microneedle pipe protruding inwardly from the inner skin so as to communicate with the space, the microneedle pipe being configured to discharge the medicinal fluid into the skin, the microneedle pipe being provided per uniform pattern, wherein the needle pipe includes an injection portion formed inclined at a predetermined angle so as to permeate the skin, the injection portion being integrally formed together with a hard support configured to support the injection portion so as to be fixed at a predetermined strength in the inner skin by injection molding in the state in which the injection portion is coupled to the support, the injection portion having a protruding length of 0.3 to 0.5 mm from the surface of the inner skin so as to permeate up to a dermis layer of the skin, and a light source portion provided on the outer skin, the light source portion including LEDs configured to irradiate light that activates cells of the skin in conjunction with the medicinal fluid, the LEDs being arranged so as to a predetermined pattern; however, the main object of this patent document is to discharge the medicinal fluid, and therefore uniform amount of light and customized irradiation are impossible, there is a high possibility of an eyeball being directly exposed to LED light, whereby serious side effects occur, and it is difficult to generate uniform parallel light described in the specification through the construction of a convex lens around the LEDs.

Patent Document 9 relates to a light guide type LED mask device including a support unit, a light emission unit, a light guide unit, a reflection unit, and a controller. Patent Document 9 provides a light guide type LED mask device configured such that LED light, such as visible light or near-infrared light, is uniformly radiated to the entirety of a facial skin, which, however, is a construction similar to Patent Document 7, and therefore this patent document has the problem encountered in Patent Document 7. In addition, nonuniform light guide may occur due to introduction of moisture or dust into the light guide unit, and it is impossible to radiate light to the face in the state in which the face is minutely divided, whereby there is a problem in that a means capable of preventing radiation of light to an eyeball is not provided.

Patent Document 10 relates to an LED mask, wherein LED light irradiation time and wavelength are automatically selected depending on skin condition of an LED mask wearer; however, side effects, such as spots, may occur on the skin due to very nonuniform irradiation when worn for a long time.

Patent Document 11 relates to an LED mask including an inner skin member formed so as to have a curved shape corresponding to a face, an inner surface of the inner skin member being attached to the face, an LED board provided at an outer surface of the inner skin member, the LED board including an LED element configured to radiate LED light to the face, and an outer skin member provided at an outer surface of the LED board. Patent Document 11 provides an LED mask configured such that the size of the LED mask can be adjusted by adjusting a gap in an incision portion depending on the size or shape of the face of the user and the LED mask can be used irrespective of place; however, there are problems in that a means capable of uniformly radiating light radiated from the LED board is not provided and a means capable of preventing radiation of light to an eyeball is weak.

Although many technologies related to LED masks have been proposed, as described above, the problems established through the present invention have never been recognized, and a solution thereto has not yet been suggested.

### (Patent Documents)

Korean Registered Patent Publication No. 10-1648415 (2016.08.09) ("Patent Document 1")
Korean Registered Patent Publication No. 10-2034145 (2019.10.14) ("Patent Document 2")
Korean Registered Patent Publication No. 10-1735703 (2017.05.08) ("Patent Document 3")
Korean Registered Patent Publication No. 10-1863698 (2018.05.28) ("Patent Document 4")
Korean Registered Patent Publication No. 10-1862280 (2018.05.23) ("Patent Document 5")
Korean Registered Patent Publication No. 10-1236335 (2013.02.18) ("Patent Document 6")
Korean Registered Patent Publication No. 10-1807533 (2017.12.05) ("Patent Document 7")
Korean Registered Patent Publication No. 10-1823263 (2018.01.24) ("Patent Document 8")
Korean Patent Application Publication No. 2019-0121715 (2019.10.28) ("Patent Document 9")
Korean Patent Application Publication No. 2019-0103077 (2019.09.04) ("Patent Document 10")
Korean Registered Patent Publication No. 10-1616707 (2016.04.25) ("Patent Document 11")
Korean Patent Application Publication No. 2019-0018383 (2019.02.22) ("Patent Document 12")

### (Non-Patent Documents)

Joel E. Pessa, "An Algorithm of Facial Aging: Verification of Lambros's Theory by Three-Dimensional Stereolithography, with Reference to the Pathogenesis of Midfacial Aging, Scleral Show, and the Lateral Suborbital Trough Deformity", PLASTIC AND RECONSTRUCTIVE SURGERY, August, p479-488, (2000)
Bryan Mendelson and Chin-Ho Wong, "Changes in the Facial Skeleton With Aging: Implications and Clinical Applications in Facial Rejuvenation ", Aesth. Plast. Surg. 36: p753-760 (2012)
David M. Kahn and Robert B. Shaw Jr., "Overview of Current Thoughts on Facial Volume and Aging", FACIAL PLASTIC SURGERY/VOLUME 26, NUMBER 5, p350-355 (2010)

### [Disclosure]

### [Technical Problem]

The present invention has been made in view of the above problems, and it is an object of the present invention to provide an LED irradiator capable of radiating uniform light to a face of a user irrespective of the shape of the face of the user and performing customized light irradiation therapy depending on position of a facial skin. It is another object of the present invention to provide an LED irradiator capable of providing an optical pattern for facial skin recovery. It is a further object of the present invention to provide an LED irradiator capable of preventing direct radiation of LED light to an eyeball and performing customized light irradiation to an infraorbital margin and a Crow's foot, on which effects by light irradiation therapy may be maximized.

### [Technical Solution]

In order to accomplish the above objects, in the present invention, a face lens is added to an LED light source such that uniform light can be radiated to a predetermined region of a face. Various variables that may affect light radiated from LEDs until the light reaches the skin, such as the distance from the skin to the light source, the curved surface of the face, change in illuminance depending on the diffusion angle of light, are compensated through the face lens. As a result, it is possible to uniformly radiate light to a specific region of the skin.

Furthermore, the face lens and at least one of a wavelength, direction, intensity, and flashing pattern of each individual LED are adjusted to provide customized light irradiation therapy depending on the position on a facial skin.

An orbital light irradiation unit directly attached to the face or disposed adjacent to the face in the vicinity of the eye is provided, wherein light is transmitted to the orbital light irradiation unit through an optical fiber, whereby it is possible to effectively remedy an infraorbital margin or a Crow's foot while the light is not directly irradiate to the eyeball.

In addition, as another method of radiating light to an infraorbital part, an eye patch unit, configured to allow light from the light source unit to be radiated only to an infraorbital periphery of the user therethrough while not being directly radiated to the eye of the user, may be added.

The eye patch unit may be configured to have any one of a doughnut structure extending in a leftward-rightward direction such that both eyes are located therein, a structure in which a partition wall is added to a middle part of the doughnut structure extending in the leftward-rightward direction, and two doughnut structures corresponding to a left eye and a right eye.

As a concrete example of the technical solution, the present invention provides an LED irradiator including a case unit configured to cover at least a part of a face of a user and a light source unit provided inside the case unit, the light source unit including a plurality of LEDs configured to radiate light to the face of the user.

The light source unit may include the plurality of LEDs and a face lens provided at a front surface of each of the plurality of LEDs, the face lens being configured to adjust at least one of a wavelength, direction, dispersion, and intensity of the light radiated to the face of the user.

The face lens may be at least one of a) a microarray lens, b) a Fresnel lens, c) a concave mirror lens having a light source disposed on a parabolic focus thereof, d) a lens including a microscopic dispersion portion disposed at a front surface of an LED light source, e) a reflection portion configured to reflect the light radiated from the LEDs, and f) a polymer material configured to disperse light.

The face lens may include at least one unit lens, wherein the unit lens may include a peripheral portion that is straight, curved, or straight and curved, at least one of overlapping, individual color, refractive index, transparency, thickness, coating, and surface roughness of the unit lens may be adjusted to adjust the amount of light transmitted through the unit lens, and at least one of the disposition direction, individual surface shape, and sectional shape of the unit lens may be adjusted to adjust at least one of the direction, intensity, dispersion, concentration, and focal distance of light.

The unit lens may be provided with a transmission portion including a hole or a slit configured to allow the light radiated from the LEDs to be diffracted therethrough or an additional reflection portion configured to reflect the light radiated from the LEDs.

Here, one LED may correspond to one unit lens, a plurality of LEDs may correspond to one unit lens, or one LED may correspond to a plurality of unit lenses.

At this time, the unit lens may have a uniform overall thickness irrespective of whether the unit lens is a concave lens or a convex lens.

The face lens may optimally adjust the diffusion angle of the light radiated from the LEDs such that symmetry of light in a predetermined region of the face of the user exceeds 50% irrespective of the distance between the LEDs and the face or may adjust the light radiated from the LEDs so as to be radiated as parallel light.

In order that symmetry of light in the predetermined region of the face of the user exceeds 50%, the light radiated from the LEDs may be radiated as parallel light, and the diffusion angle of the light may be adjusted to +10 degrees to -10 degrees based on the parallel light.

At least one of the wavelength, energy, and intensity of the light radiated to the face of the user is changed by an upper part of the face, which is above an eyebrow, a middle part of the face, which is from a part under the eyebrow, including the eyebrow, to a part above a lip, and a part under the face, which is under the lip or by a forehead, an infraorbital margin, a Crow's foot, an eyeball, a nasolabial fold, and other parts.

The light radiated from the light source unit may form a wave irradiation pattern by adjusting at least one of the wavelength, direction, intensity, and flashing pattern of each individual LED.

In connection with eyeball protection, a screening unit configured to prevent direct radiation of the light radiated from the light source unit to the eye of the user may be added. The screening unit may be made of an elastic material configured to reflect or absorb light at a surface thereof. Specifically, the screening unit may include a cover portion configured to cover a periphery of the eye, the cover portion being made of an elastic material, and a cover fixing portion configured to push the cover portion from an outer surface of the cover portion such that the cover portion and the face of the user at the periphery of the eye are brought into tight contact with each other.

As another solution in connection with eyeball protection, the LED irradiator may further include 1) an orbital light irradiation unit directly attached to the face or disposed adjacent to the face on at least a part of the face, at least a part of the orbital light irradiation unit being transparent, and 2) an orbital light source unit configured to provide light to the orbital light irradiation unit.

The orbital light irradiation unit may be 1) a structure configured such that at least a part of a surface of the orbital light irradiation unit that abuts the skin is transparent and the other surfaces of the orbital light irradiation unit are opaque or are configured to prevent exposure of light to the outside or 2) an optical fiber connected to the orbital light source unit.

The orbital light source unit may provide light to the orbital light irradiation unit using at least one of 1) an LED disposed adjacent to the orbital light irradiation unit, 2) an LED inserted into and disposed in the orbital light irradiation unit, and 3) an LED disposed spaced apart from the orbital light irradiation unit and an optical fiber or a light guide configured to connect the LED and the orbital light irradiation unit to each other.

The light radiated to the orbital light irradiation unit may be radiated only to the face of the user around the eye. At this time, the light radiated from the orbital light irradiation unit may form a pattern, or the amount or color of the light may be changed over time.

As a further solution for eyeball protection or radiation of light only to the infraorbital margin, an eye patch unit configured to allow the light from the light source unit to be radiated only to the infraorbital periphery of the user therethrough while not being directly radiated to the eye of the user, may be added.

The eye patch unit may be configured to have any one of a doughnut structure extending in a leftward-rightward direction such that both eyes are located therein, a structure in which a partition wall is added to a middle part of the doughnut structure extending in the leftward-rightward direction, and two doughnut structures corresponding to a left eye and a right eye.

The eye patch unit may include a light source blocking portion located in the middle of the doughnut structure, the light source blocking portion being configured to block the light from the light source unit, and an outer circumferential portion continuously connected to the light source blocking portion, the outer circumferential portion including an infraorbital irradiation portion configured to radiate the light from the light source unit to at least a part of an infraorbital skin of the user.

The middle of the doughnut structure may be open to the outside, may be hidden by the case unit, or may be hidden by a separate screen.

The inside or the outside of the light source blocking portion may include a material configured to prevent transmission of light therethrough, and the infraorbital irradiation portion may radiate the light from the light source unit in a state of directly contacting the infraorbital skin of the user or in a state of not contacting the infraorbital skin of the user. Each of the light source blocking portion and the infraorbital irradiation portion may be made of an elastic material since the light source blocking portion and the infraorbital irradiation portion contact the skin or are located adjacent to the skin.

When the LED irradiator is used once, the total energy of light radiated from the light source unit to 1 cm² of the face of the user may be 50 J or less, preferably 25 J or less, more preferably 15 J or less, and the maximum intensity of the light radiated from the light source unit to 1 cm² of the face of the user may be 200 mW or less, preferably 100 mW or less, more preferably 50 mW or less. Meanwhile, in order to achieve a desired effect of the present invention, the minimum intensity of the light radiated from the light source unit to 1 cm² of the face of the user must be 1 mW or more. The total energy and intensity (mW) of light radiated from the light source unit to the face of the user may be changed depending on the position on the face of the user.

The LED according to the present invention may include at least one of a visible LED, an infrared LED, and an ultraviolet LED.

The following effects may be achieved depending on the wavelength of the LED. The blue wavelength of the LED, which is 440 to 500 nm, may be used to remove Propionibacterium acne, to reduce acne inflammation, and to adjust sebaceous gland secretion, and also be used as alternative treatment to eczema or psoriasis through non-UV irradiation.

The red wavelength of the LED, which is 625 to 700 nm, has effects of increasing natural moisturizing power, alleviating flushing and inflammation, reducing pores, adjusting sebaceous gland, improving blood circulation, treating skin injury. As a concrete example, rosacea may be remedied.

Infrared light has a wavelength of 700 to 1000 nm, which is longest. Since the infrared light permeates a deep layer of the skin so as to be absorbed thereby due to the long wavelength thereof, the infrared light is effective to remedy wrinkles, to improve skin elasticity, and to reduce inflammation and related pain. In addition, the infrared light effectively treats pustules.

The case unit according to the present invention may include a fixing portion configured to allow the plurality of LEDs to be fixed thereto, wherein the fixing portion may be a rib-shaped frame. An outer skin unit made of a transparent material may be disposed at the outermost side of the case unit, and the material for the outer skin unit may be any one of various resins, and may include at least one of the following resins.

Polypropylene (PP), polyethylene (PE), polyamide (PA), polystyrene (PS), polyester (PES), polycarbonate (PC), thermoplastic polyurethane (TPU), polyacetal (POM), polymethyl methacrylate (PMMA), polyethylene terephthalate (PET), polybutylene terephthalate (PBT), acrylonitrile butadiene styrene (ABS), polyetherimide (PEI), polyetheretherketone (PEEK), polyphenylene sulfide (PPS), polylactic acid (PLA), polyvinyl chloride (PVC), modified polyphenylene oxide (MPPO), thermoplastic elastomer (TPE), and polyvinyl acetate (PVA).

The present invention may provide arbitrary combinations of the above solving means.

### [Advantageous Effects]

The present invention has the following effects comparable to a conventional LED irradiator.

First, it is possible to provide uniform amount of light to a face. In the present invention, a uniform amount of light radiated from an LED light source is provided to the face of the user through a face lens including a plurality of unit lenses. The diffusion angle of light that has passed through the face lens is optimally adjusted, or the light is radiated in parallel, depending on the distance between the light source and the skin, whereby it is possible to provide a uniform amount of light.

Second, it is possible to perform customized light irradiation therapy depending on position of a facial skin. In the present invention, the face of the user may be divided into an upper part of the face, which is above an eyebrow, a middle part of the face, which is from a part under the eyebrow, including the eyebrow, to a part above a lip, and a part under the face, which is under the lip, or may be divided into a forehead, an infraorbital margin, a Crow's foot, an eyeball, a nasolabial fold, and other parts. At least one of the wavelength, energy, and intensity of the light radiated to the face may be changed depending on the above divided parts.

Third, the present invention provides a separate irradiation method capable of preventing direct radiation of light from the light source to an eyeball and being directly applied to the infraorbital margin. As a result, the eyeball of the user may be protected, and the infraorbital margin, which is greatly affected by light irradiation therapy, may be remarkably remedied. In the present invention, a screening unit configured to protect the eye of the user may be added, or an orbital light irradiation unit and an orbital light source unit and/or an eye patch unit, each of which is configured to allow LED light to be directly radiated to the skin around the eyeball therethrough while not being directly radiated to the eye of the user, may be separately provided.

### [Description of Drawings]

FIG. 1 is a view showing the anatomy of the skull for describing the circumference of an eyeball.
FIG. 2 is a perspective view of an LED irradiator according to an embodiment of the present invention.
FIG. 3 is an exploded perspective view showing only a face lens, a fixing portion, and an outer skin unit of the LED irradiator according to the embodiment of the present invention.
FIG. 4 is a schematic view showing a face lens according to a first embodiment of the present invention and an example in which LEDs are disposed with respect thereto.
FIG. 5 is a schematic view showing a face lens according to a second embodiment of the present invention and an example in which LEDs are disposed with respect thereto.
FIG. 6 is a schematic view showing a face lens according to a third embodiment of the present invention and an example in which LEDs are disposed with respect thereto.
FIG. 7 is a schematic view of unit lenses of a face lens according to a fourth embodiment of the present invention.
FIG. 8 is a schematic view of unit lenses of a face lens according to a fifth embodiment of the present invention.
FIG. 9 is a schematic view showing an orbital light irradiation unit and an orbital light source unit according to an embodiment of the present invention.
FIG. 10 is a schematic view showing an orbital light irradiation unit and an orbital light source unit according to another embodiment of the present invention.
FIG. 11 is a schematic view showing an orbital light irradiation unit and an orbital light source unit according to a further embodiment of the present invention.
FIG. 12 is a schematic view of an eye patch unit according to an embodiment of the present invention.
FIG. 13 is a schematic view showing the section of the eye patch unit according to the embodiment of the present invention.
FIGS. 14 to 17 are schematic views showing only the eye patch unit according to the embodiment of the present invention when a light blocking portion of the eye patch unit is and when the light blocking portion of the eye patch unit is not removed.

### [Best Mode]

Now, preferred embodiments of the present invention will be described in detail with reference to the accompanying drawings such that the preferred embodiments of the present invention can be easily implemented by a person having ordinary skill in the art to which the present invention pertains. In describing the principle of operation of the preferred embodiments of the present invention in detail, however, a detailed description of known functions and configurations incorporated herein will be omitted when the same may obscure the subject matter of the present invention.

In addition, the same reference numbers will be used throughout the drawings to refer to parts that perform similar functions or operations. In the case in which one part is said to be connected to another part in the entire specification, not only may the one part be directly connected to the other part, but also, the one part may be indirectly connected to the other part via a further part. In addition, that a certain element is included does not mean that other elements are excluded, but means that such elements may be further included unless mentioned otherwise.

Hereinafter, the present invention will be described in more detail with reference to embodiments. However, the embodiments are provided merely to illustratively describe the present invention, and therefore the scope of the present invention is not limited by the embodiments.

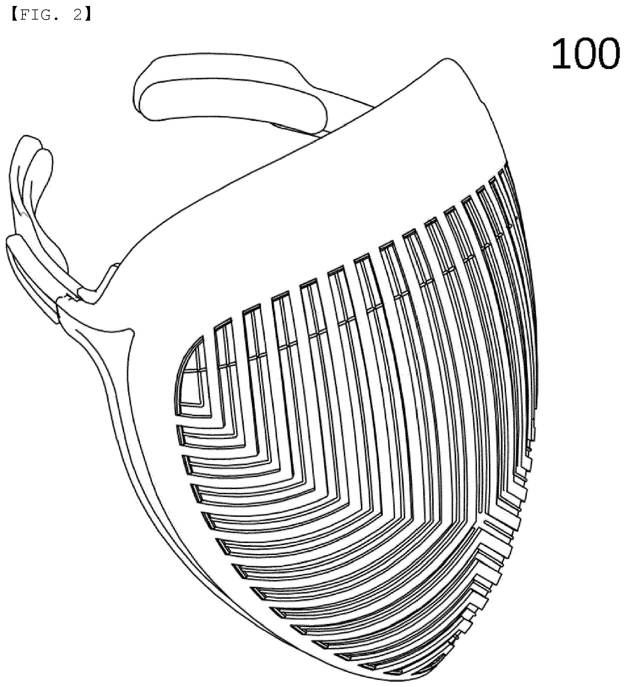

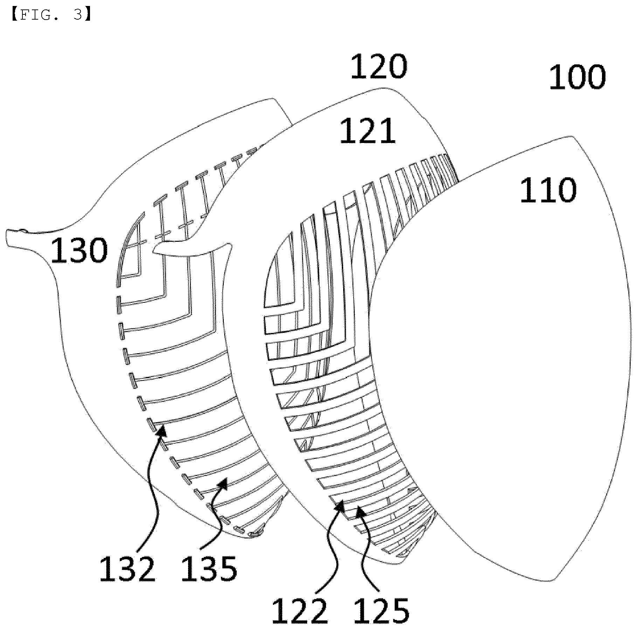

Now, a description will be given with reference to FIGS. 2 to 17. FIG. 2 is a perspective view of an LED irradiator 100 according to an embodiment of the present invention, and FIG. 3 is an exploded perspective view showing only a face lens 130, a case unit 120 including a fixing portion 121, and an outer skin unit 110 of the LED irradiator 100 according to the embodiment of the present invention.

The present invention provides an LED irradiator 100 including a case unit 120 configured to cover at least a part of a face of a user and a light source unit 140, 240, or 340 provided inside the case unit 120, the light source unit including a plurality of LEDs configured to irradiate the face of the user with light.

The light source unit 140, 240, or 340 includes the plurality of LEDs and a face lens 130 provided at a front surface of each of the plurality of LEDs, the face lens being configured to adjust at least one of the wavelength, direction, and intensity of the light radiated to the face of the user.

In FIG. 3, the outer skin unit 110 may be disposed at the outermost side and may be made of a transparent material. Various kinds of resins may be used as the material for the outer skin unit 110, which may include at least one of the following resins.

Polypropylene (PP), polyethylene (PE), polyamide (PA), polystyrene (PS), polyester (PES), polycarbonate (PC), thermoplastic polyurethane (TPU), polyacetal (POM), polymethyl methacrylate (PMMA), polyethylene terephthalate (PET), polybutylene terephthalate (PBT), acrylonitrile butadiene styrene (ABS), polyetherimide (PEI), polyetheretherketone (PEEK), polyphenylene sulfide (PPS), polylactic acid (PLA), polyvinyl chloride (PVC), modified polyphenylene oxide (MPPO), thermoplastic elastomer (TPE), and polyvinyl acetate (PVA).

The fixing portion 121 according to FIG. 3 may include rib-shaped frames 125 and a space 122 defined between the frames. The fixing portion 121 may also be made of a resin or a metal. A metal coated with an insulative resin may be used.

The face lens 130 according to FIG. 3 may include unit lenses 135 and a space 132 defined between the unit lenses 135 so as to partition the unit lenses from each other.

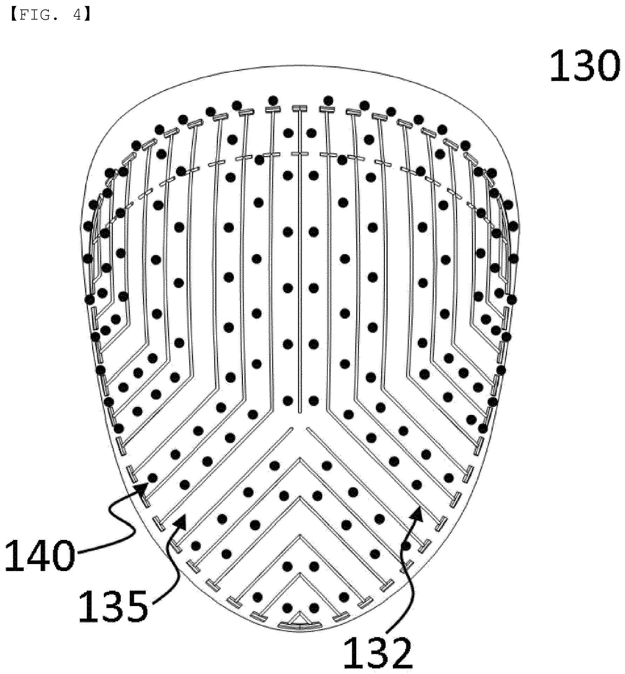

FIG. 4 is a schematic view showing a face lens 130 according to a first embodiment of the present invention and an example in which LEDs are disposed with respect thereto. The face lens 130 may include unit lenses 135 and a space 132 defined between the unit lenses 135 so as to partition the unit lenses from each other. Each individual LED of the light source unit 140 is fixed to the fixing portion 121 disposed at a front surface of the face lens 130. When the fixing portion 121 and the face lens 130 are coupled to each other, however, individual LEDs of the light source unit 140 may be disposed as shown in FIG. 4.

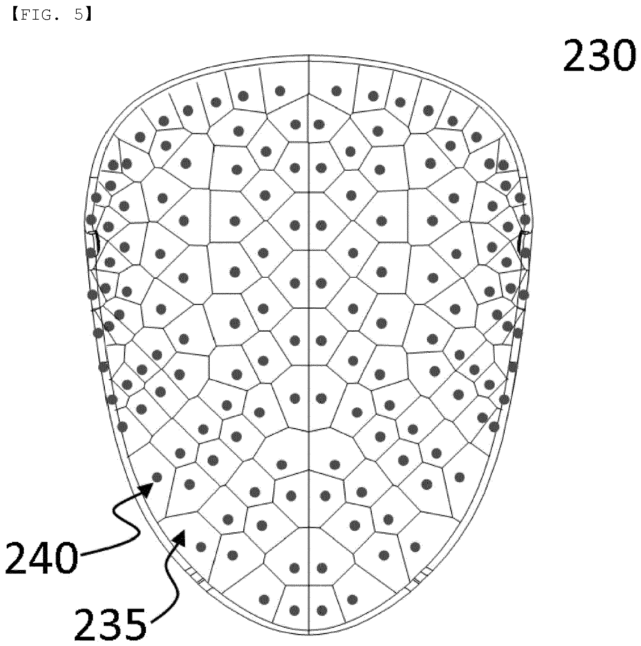

FIG. 5 is a schematic view showing a face lens 230 according to a second embodiment of the present invention and an example in which LEDs are disposed with respect thereto. In the face lens 230 according to FIG. 5, unit lenses 235 are disposed without gaps therebetween so as to form a latticed structure. In FIG. 5, one LED is disposed at one unit lens 235. Alternatively, however, one LED may correspond to one unit lens, a plurality of LEDs may correspond to one unit lens, or one LED may correspond to a plurality of unit lenses.

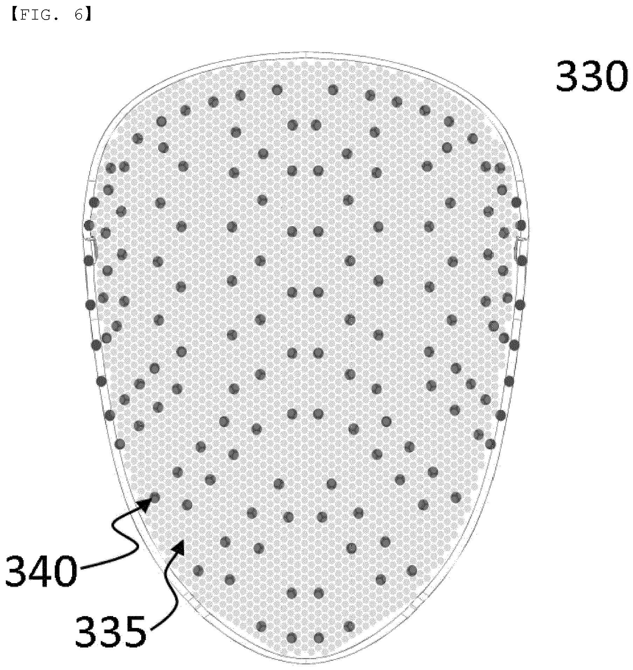

FIG. 6 is a schematic view showing a face lens 330 according to a third embodiment of the present invention and an example in which LEDs are disposed with respect thereto. In the face lens 330 according to FIG. 6, unit lenses 335 are disposed without gaps therebetween so as to form a latticed structure.

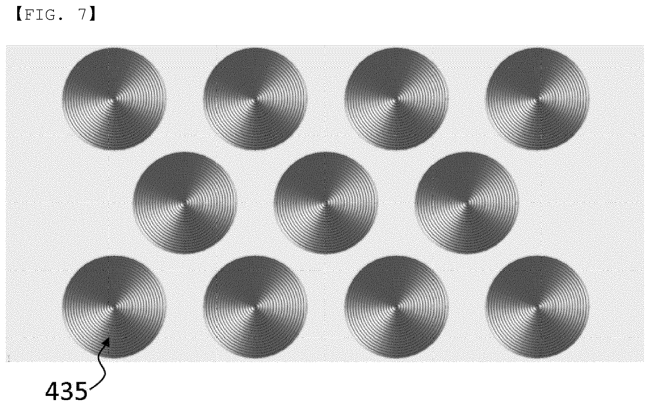

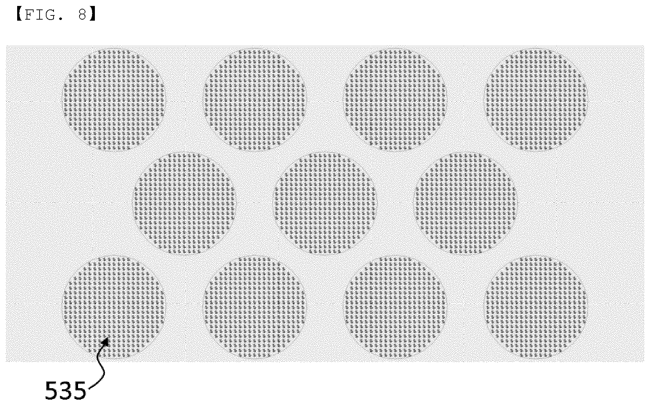

FIGS. 7 and 8 are schematic views of unit lenses of face lenses according to fourth and fifth embodiments of the present invention, respectively.

Each unit lens may be a circular Fresnel lens, as shown in FIG. 7, or may be a microarray lens, as shown in Fig. 8. The microarray lens, which is a single large lens constituted by a plurality of gathered microlenses, is a lens formed like an insect eye, particularly a dragonfly eye.

Although not shown in the drawings, the face lens according to the present invention may be made of one polymer material that simply disperses light. Similarly to the conventional art in which a glass surface of an incandescent lamp is coated so as to be milky such that light is dispersed, the inside of the face lens may be coated, or the face lens may be made of a semitransparent material.

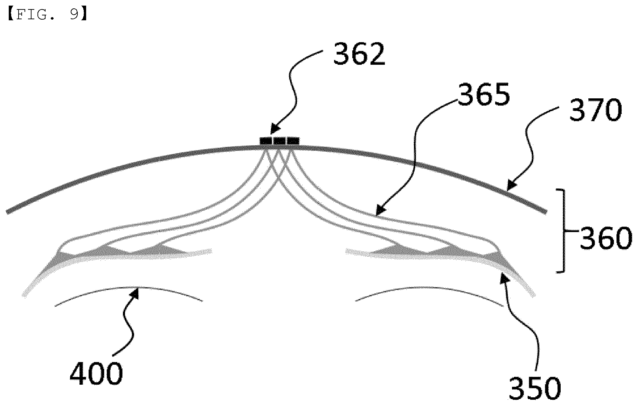

FIGS. 9 to 11 are schematic view respectively showing orbital light irradiation units and orbital light source units according to various embodiments of the present invention.

The LED irradiator further includes 1) an orbital light irradiation unit 150, 250, or 350 directly attached to the face or disposed adjacent to the face in the vicinity of an eye 400 of the user, at least a part of the orbital light irradiation unit being transparent, and 2) an orbital light source unit 160, 260, or 360 configured to provide light to the orbital light irradiation unit 150, 250, or 350.

At least a part of the surface of the orbital light irradiation unit 150, 250, or 350 that contacts the skin must be transparent, and the other surface must be opaque or configured to prevent exposure of light to the outside.

The orbital light source unit may be constituted by 1) LEDs 162 disposed adjacent to the orbital light irradiation unit 150, 2) LEDs 262 inserted into and disposed in the orbital light irradiation unit 250, or 3) LEDs 362 disposed spaced apart from the orbital light irradiation unit 350 and an optical fiber or a light guide 365 configured to connect the LEDs 362 to the orbital light irradiation unit 350. These may be fixed by an orbital light fixing portion 170, 270, or 370, and the orbital light fixing portion 170, 270, or 370 may not be separately provided but may be substituted by the face lens or the fixing portion.

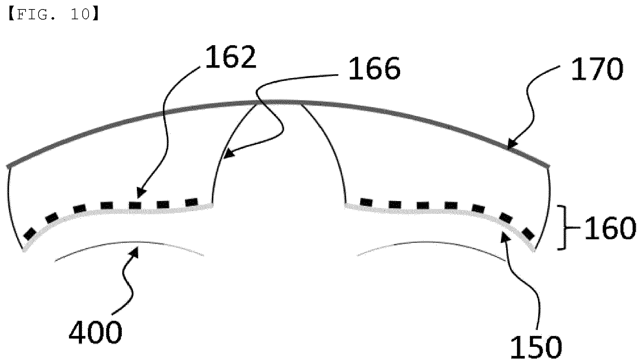

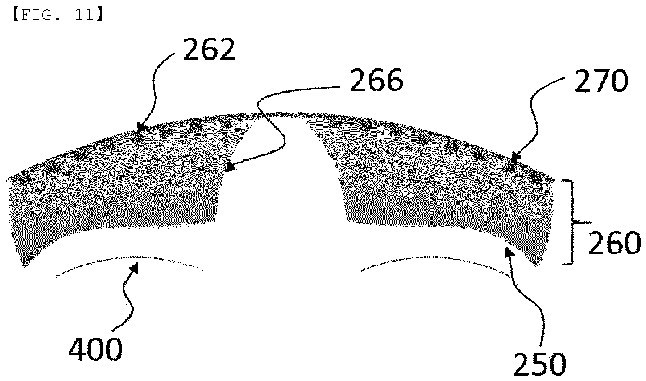

An orbital light supporting portion 166 or 266 may be provided to support the orbital light irradiation unit, or the optical fiber or the light guide 365 may also serve as the orbital light supporting portion. FIG. 10 shows that an empty space is provided between the orbital light irradiation unit 150 and the orbital light fixing portion 170, and FIG. 11 shows that an orbital light screen 266 is provided to block light.

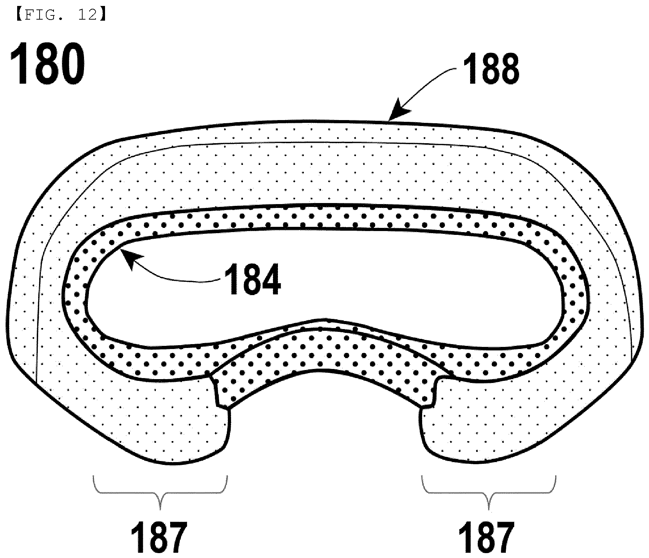

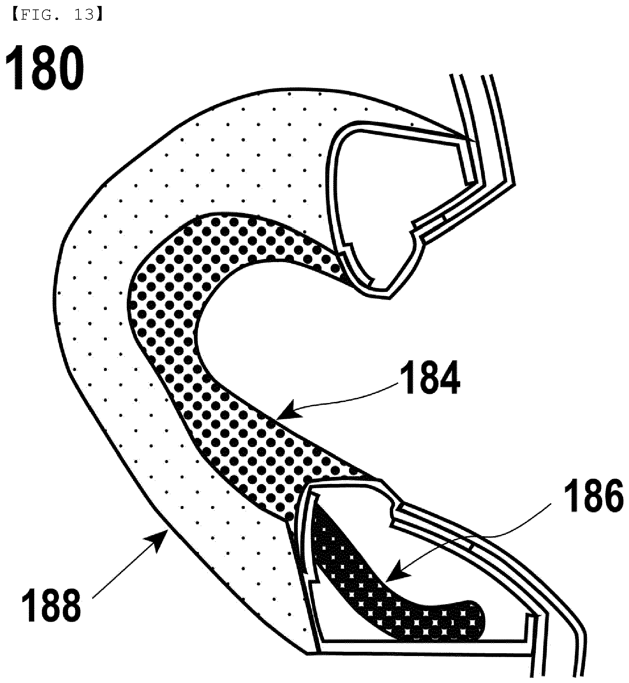

FIG. 12 is a schematic view of an eye patch unit 180 according to an embodiment of the present invention, and FIG. 13 is a schematic view showing the section of the eye patch unit 180 according to the embodiment of the present invention.

Referring to FIGS. 12 and 13, the eye patch unit 180 is configured to have a doughnut structure extending in a leftward-rightward direction such that both eyes are located therein, wherein light from the light source unit is radiated only to the infraorbital periphery of the user while not being directly radiated to eyes of the user. The eye patch unit 180 includes a light source blocking portion 184 located in the middle of the doughnut extending in the leftward-rightward direction, the light source blocking portion being configured to block light from the light source unit while opening the eyes of the user to the outside, and an outer circumferential portion 188 continuously connected to the light source blocking portion 184, the outer circumferential portion including an infraorbital irradiation portion 187 configured to radiate light from the light source unit to at least a part of an infraorbital skin of the user.

The part that is open to the outside may be hidden by a separate means.

An optical fiber configured to provide light to the infraorbital irradiation portion 187 or a resin configured to transmit light is disposed in the eye patch unit 180.

Each of the light source blocking portion 184 and the outer circumferential portion 188 may be made of an elastic material, preferably a material that weakly stimulates or does not stimulate human skin when being brought into contact therewith. Each of the light source blocking portion 184 and the outer circumferential portion 188 may be made of a semitransparent material. In particular, the light source blocking portion 184 may be generally made of a semitransparent material, and the part that faces the eyes of the user may be made of an opaque material or metal foil may be added to the inside thereof so as to be opaque.

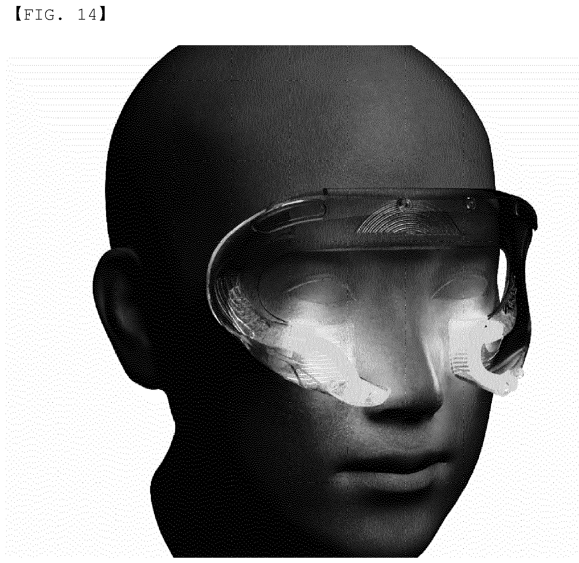

FIGS. 14 to 17 are schematic views showing only the eye patch unit according to the embodiment of the present invention when a light blocking portion of the eye patch unit is removed and when the light blocking portion of the eye patch unit is not removed.

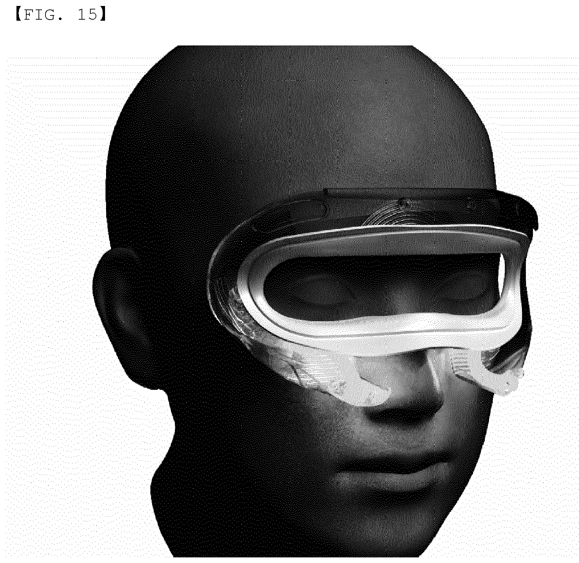

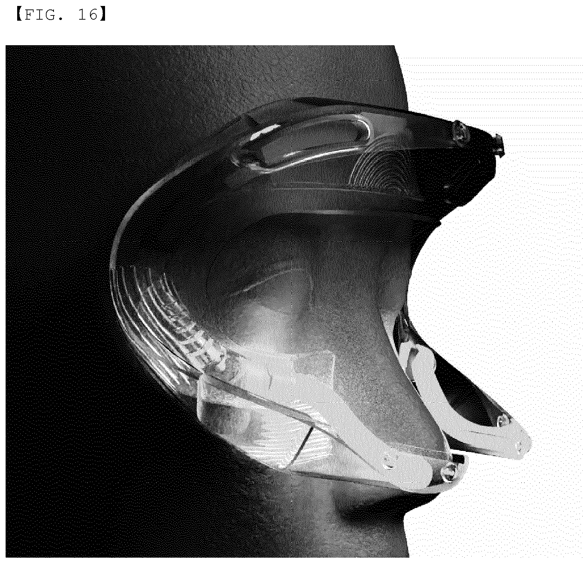

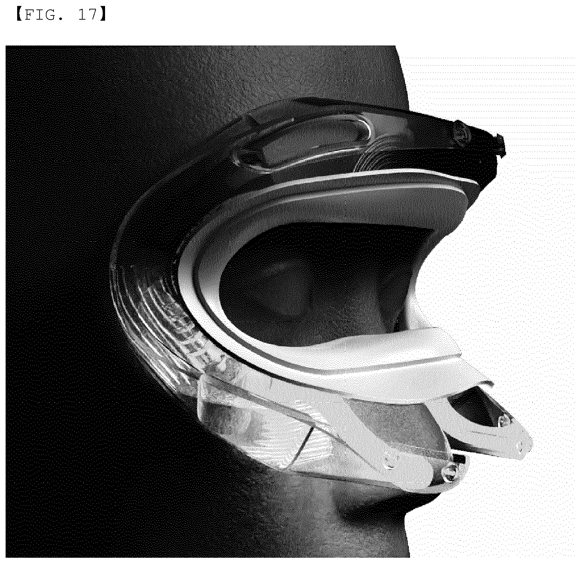

FIGS. 14 and 16 show the eye patch unit 180 in the state in which there is no light source blocking portion 184, and FIGS. 15 and 17 show the eye patch unit 180 in the state in which the light source blocking portion 184 is mounted. When there is no light source blocking portion 184, it can be seen that light is also directly radiated to the eyes of the user, whereby the vicinity of each of the eyes is bright. In the state in which the light source blocking portion 184 is mounted, the eyes and the vicinity thereof are dark.

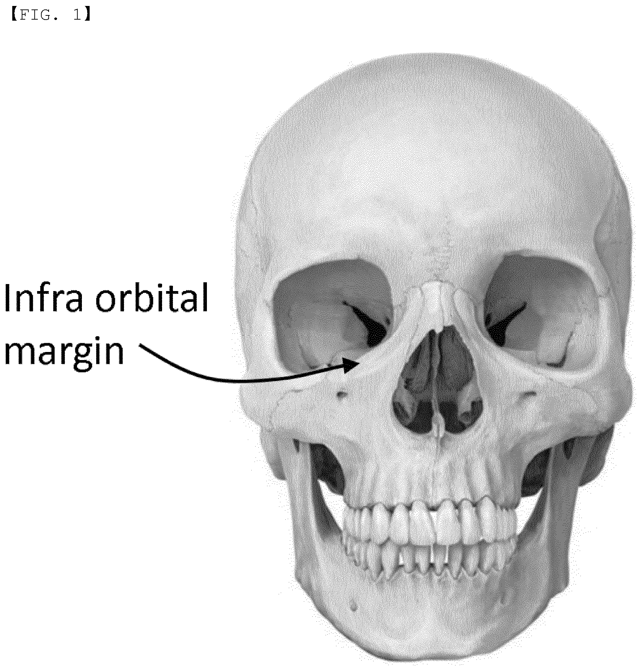

In Patent Document 12, a face contact unit 242 configured to protect each eye such that light is not directly radiated to the eye, such as swimming goggles, is provided. In Patent Document 12, the face contact unit 242 surrounds the eyes in a circle while also contacting the skin. One of the very important functions of the LED irradiator according to the present invention is to remedy the infraorbital margin; however, the construction disclosed in Patent Document 12 has a problem in that the face contact unit 242, which may be made of rubber or silicone while having an opaque color, contacts the skin at the part at which the infraorbital margin located very close to the eye (the infraorbital margin and the periphery thereof shown in FIG. 1 of the present invention) is located, whereby no light is radiated to the region.

In the present invention, the light source blocking portion 184 and the outer circumferential portion 188 are provided in order to solve the above problem while light is not directly radiated to the eye, thereby guaranteeing safety. In particular, the light source blocking portion 184 may not directly contact the skin. The minimum angle is necessary for light to be radiated to the eye. When the light source blocking portion 184 contacts the skin or is located very close to the skin, however, internal light is not directly radiated to the eye. In addition, the light source blocking portion 184 is made of a material configured to prevent transmission of light through the part located closest to the eye around the eye. Furthermore, the light source blocking portion 184 is not disposed perpendicular to the skin but is disposed inclined outwards so as to have open feeling around the eye such that the infraorbital irradiation portion 187 is disposed as close to the eye as possible, whereby the infraorbital margin remedy effect is improved.

It can be seen from FIGS. 14 to 17 that it is possible for the eye patch unit 180 according to the present invention to prevent direct radiation of light to the eye, thereby guaranteeing safety, and to clearly recognize that light is radiated to the infraorbital margin.

### (Description of Reference Numerals)

100: LED irradiator
110: Outer skin unit
120: Case unit
121: Fixing portion
122: Space between frames
125: Frame
130, 230, 330: Face lenses
132: Space between unit lenses
135, 235, 335, 435, 535: Unit lenses
140, 240, 340: Light source units
150, 250, 350: Orbital light irradiation units
160, 260, 360: Orbital light source units
162, 262, 362: LEDs
166: Orbital light supporting portion
266: Orbital light screen
365: Optical fiber or light guide
170, 270, 370: Orbital light fixing portions
180: Eye patch unit
184: Light source blocking portion
186: Eye patch unit light source
187: Infraorbital irradiation portion
188: Outer circumferential portion
400: Eye of user

### [Industrial Applicability]

The present invention relates to an LED irradiator capable of adjusting the direction and intensity of light radiated from an LED light source, and therefore the present invention is industrially applicable.

## Claims

1. An LED irradiator comprising:
a case unit configured to cover at least a part of a face of a user; and
a light source unit provided inside the case unit, the light source unit comprising a plurality of LEDs configured to irradiate the face of the user with light.

2. The LED irradiator according to claim 1, wherein the light source unit comprises:
the plurality of LEDs; and
a face lens provided at a front surface of each of the plurality of LEDs, the face lens being configured to adjust at least one of a wavelength, direction, dispersion, and intensity of the light radiated to the face of the user.

3. The LED irradiator according to claim 2, wherein the face lens is at least one of:
a) a microarray lens;
b) a Fresnel lens;
c) a concave mirror lens having a light source disposed on a parabolic focus thereof;
d) a lens comprising a microscopic dispersion portion disposed at a front surface of an LED light source;
e) a reflection portion configured to reflect the light radiated from the LEDs; and
f) a polymer material configured to disperse light.

4. The LED irradiator according to claim 2, wherein
the face lens comprises at least one unit lens,
the unit lens comprises a peripheral portion that is straight, curved, or straight and curved,
at least one of overlapping, individual color, refractive index, transparency, thickness, coating, and surface roughness of the unit lens is adjusted to adjust an amount of light transmitted through the unit lens, and
at least one of a disposition direction, individual surface shape, and sectional shape of the unit lens is adjusted to adjust at least one of a direction, intensity, dispersion, concentration, and focal distance of light.

5. The LED irradiator according to claim 4, wherein the unit lens is provided with a transmission portion comprising a hole or a slit configured to allow the light radiated from the LEDs to be diffracted therethrough or an additional reflection portion configured to reflect the light radiated from the LEDs.

6. The LED irradiator according to claim 4, wherein
one LED corresponds to one unit lens,
a plurality of LEDs corresponds to one unit lens, or
one LED corresponds to a plurality of unit lenses.

7. The LED irradiator according to claim 4, wherein the unit lens has an overall uniform thickness irrespective of whether the unit lens is a concave lens or a convex lens.

8. The LED irradiator according to claim 2, wherein the face lens adjusts a diffusion angle of the light radiated from the LEDs such that symmetry of light in a predetermined region of the face of the user exceeds 50% irrespective of a distance between the LEDs and the face or adjusts the light radiated from the LEDs so as to be radiated as parallel light.

9. The LED irradiator according to claim 1, wherein at least one of a wavelength, energy, and intensity of the light radiated to the face of the user is changed:
by an upper part of the face, which is above an eyebrow, a middle part of the face, which is from a part under the eyebrow, including the eyebrow, to a part above a lip, and a part under the face, which is under the lip; or
by a forehead, an infraorbital margin, a Crow's foot, an eyeball, a nasolabial fold, and other parts.

10. The LED irradiator according to claim 1, wherein the light radiated from the light source unit forms a wave irradiation pattern by adjusting at least one of a wavelength, direction, intensity, and flashing pattern of each individual LED.

11. The LED irradiator according to claim 1, further comprising a screening unit configured to prevent direct irradiation of an eye of the user with the light radiated from the light source unit.

12. The LED irradiator according to claim 11, wherein the screening unit is made of an elastic material configured to reflect or absorb light at a surface thereof.

13. The LED irradiator according to claim 11, wherein the screening unit comprises:
a cover portion configured to cover a periphery of the eye, the cover portion being made of an elastic material; and
a cover fixing portion configured to push the cover portion from an outer surface of the cover portion such that the cover portion and the face of the user at the periphery of the eye are brought into tight contact with each other.

14. The LED irradiator according to claim 1, further comprising:
1) an orbital light irradiation unit directly attached to the face or disposed adjacent to the face on at least a part of the face, at least a part of the orbital light irradiation unit being transparent; and
2) an orbital light source unit configured to provide light to the orbital light irradiation unit.

15. The LED irradiator according to claim 14, wherein the orbital light irradiation unit is:
1) a structure configured such that at least a part of a surface of the orbital light irradiation unit that abuts a skin is transparent and the other surfaces of the orbital light irradiation unit are opaque or are configured to prevent exposure of light to an outside; or
2) an optical fiber connected to the orbital light source unit.

16. The LED irradiator according to claim 14, wherein the orbital light source unit provides light to the orbital light irradiation unit using at least one of:
1) an LED disposed adjacent to the orbital light irradiation unit;
2) an LED inserted into and disposed in the orbital light irradiation unit; and
3) an LED disposed spaced apart from the orbital light irradiation unit and an optical fiber or a light guide configured to connect the LED and the orbital light irradiation unit to each other.

17. The LED irradiator according to claim 14, wherein the light radiated to the orbital light irradiation unit is radiated only to the face of the user around the eye.

18. The LED irradiator according to claim 1, further comprising:
an eye patch unit configured to have any one of:
a doughnut structure extending in a leftward-rightward direction such that both eyes are located therein;
a structure in which a partition wall is added to a middle part of the doughnut structure extending in the leftward-rightward direction; and
two doughnut structures corresponding to a left eye and a right eye, wherein
light from the light source unit is radiated only to an infraorbital periphery of the user while not being directly radiated to the eyes of the user.

19. The LED irradiator according to claim 18, wherein the eye patch unit comprises:
a light source blocking portion located in a middle of the doughnut structure, the light source blocking portion being configured to block the light from the light source unit; and
an outer circumferential portion continuously connected to the light source blocking portion, the outer circumferential portion comprising an infraorbital irradiation portion configured to irradiate the light from the light source unit to at least a part of an infraorbital skin of the user.

20. The LED irradiator according to claim 19, wherein
an inside or an outside of the light source blocking portion comprises a material configured to prevent transmission of light therethrough, and
the infraorbital irradiation portion irradiates the light from the light source unit in a state of directly contacting the infraorbital skin of the user or in a state of not contacting the infraorbital skin of the user.

21. The LED irradiator according to claim 1, wherein
when the LED irradiator is used once, a total energy of light radiated from the light source unit to 1 cm² of the face of the user is 50 J or less, and
a maximum intensity of the light radiated from the light source unit to 1 cm² of the face of the user is 200 mW or less .

22. The LED irradiator according to claim 1, wherein a total energy and intensity (mW) of light radiated from the light source unit to the face of the user are changed depending on position of face of the user.

23. The LED irradiator according to claim 1, wherein each of the LEDs comprises at least one of a visible LED, an infrared LED, and an ultraviolet LED.

24. The LED irradiator according to claim 1, wherein the case unit comprises a fixing portion configured to allow the plurality of LEDs to be fixed thereto.

25. The LED irradiator according to claim 24, wherein the fixing portion is a rib-shaped frame.

26. The LED irradiator according to claim 1, further comprising an outer skin unit disposed at an outermost side of the case unit, the outer skin unit being made of a transparent material.

27. The LED irradiator according to claim 26, wherein the material for the outer skin unit comprises at least one of polypropylene (PP), polyethylene (PE), polyamide (PA), polystyrene (PS), polyester (PES), polycarbonate (PC), thermoplastic polyurethane (TPU), polyacetal (POM), polymethyl methacrylate (PMMA), polyethylene terephthalate (PET), polybutylene terephthalate (PBT), acrylonitrile butadiene styrene (ABS), polyetherimide (PEI), polyetheretherketone (PEEK), polyphenylene sulfide (PPS), polylactic acid (PLA), polyvinyl chloride (PVC), modified polyphenylene oxide (MPPO), thermoplastic elastomer (TPE), and polyvinyl acetate (PVA).
